# EUROPEAN PATENT SPECIFICATION

(11) **EP 0 851 756 B1**
(45) Date of publication and mention of the grant of the patent: **15.01.2003**
(21) Application number: 96929991.6
(22) Date of filing: 16.09.1996
(51) Int. Cl.: A61K 31/415, A61K 31/00, A61P 25/14

(54) **USE OF ONDANSETRON IN THE MANUFACTURE OF A MEDICAMENT FOR THE TREATMENT OF TREMOR**
DIE VERWENDUNG VON ONDANSETRON ZUR HERSTELLUNG EINES MEDIKAMENTES ZUR BEHANDLUNG VON TREMOR
UTILISATION D' ONDANSETRON POUR LA FABRICATION D' UNE MEDICAMENT POUR LE TRAITMENT DE TREMULATION

(30) Priority: 18.09.1995 GB 9519021; 18.09.1995 GB 9519029
(43) Date of publication of application: 08.07.1998
(73) Proprietor: GLAXO GROUP LIMITED, Greenford, Middlesex UB6 0NN (GB)
(72) Inventor: RICE, George, Peter, Arthur, London, Ontario N5X 3W2 (CA)
(74) Representative: Lane, Graham Mark Hamilton
(86) International application number: CA9600616
(87) International publication number: WO97010823

(56) References cited:
- EP-A- 0 337 547
- EP-A- 0 407 137
- EP-A- 0 591 027
- EP-A- 0 646 583
- EP-A- 0 732 334
- FR-A- 2 674 853
- FR-A- 2 693 194
- GB-A- 2 229 182
- J.NEURAL.TRANSM.GEN.SECT., vol. 86, no. 3, 1991, pages 223-228, XP000616560 K.A.YOUNG ET AL.: "Subchronic buspirone, mesulergine, and ICS 205-930 lack effects on D1 and D2 dopamine binding in the rat striatum during chronic haloperidol treatment"
- DIALOG INFORMATION SERVICES, FILE 73: EMBASE, ACCESSION NUMBER 6436035, XP002024612 & PSYCHOPHARMACOLOGY, vol. 92, no. 4, 1987, pages 520-523, G.T.SHEARMAN ET AL.: "Effect of the selective 5-HTsub 3 receptor antagonists ICS 205-930 and MDL 72222 on 5-HTP-induced head shaking and behavioral symptoms induced by 5-methoxy-N,N,dimethyltryptamine in rats: Comparison with some other 5-HT receptor antagonists"
- NAUNYN-SCHMIEDEBERG'S ARCH. PHARMACOL., vol. 346, no. 1, 1992, pages 12-19, XP000616538 H.NISSBRANDT ET AL.: "The influence of serotoninergic drugs on dopaminergic neurotransmission in rat substantia nigra, striatum and limbic forebrain in vivo"
- PHARMACOLOGY BIOCHEMISTRY AND BEHAVIOR, vol. 52, no. 3, 1995, pages 493-501, XP000616549 S.K.YEGHIAYAN ET AL: "Serotonergic Stimulation of the Ventrolateral Striatum Induces Orofacial Stereotypy"
- LANCET, vol. 345, 6 May 1995, pages 1182-1183, XP000644332 G.P.A.RICE ET AL.: "Ondansetron for intractable vertigo complicating acute brainstem disorders"
- ANN.NEUROL., vol. 38, no. 6, December 1995, page 973 XP000644312 G.RICE ET AL.: "Ondansetron for Disabling Cerebellar Tremor"

## Description

The invention relates to the use of ondansetron or a pharmaceutically acceptable derivative thereof in the manufacture of a medicament for the treatment of tremor.

5-HT₃ receptor antagonists may be identified by methods well known in the art, for example by their ability to inhibit 3-(5-methyl-1H-imidazole-4-yl)-1-[1-[³H]-methyl-1H-indol-3-yl]-1-propanone binding in rat entorhinal cortex homogenates (following the general procedure described by G Kilpatrick *et al*, Nature, 1987, 330, 746-748), and/or by their effect on the 5-HT-induced Bezold-Jarisch (B-J) reflex in the cat (following the general method described by A Butler *et al*, Br. J. Pharmacol., 94, 397-412 (1988)).

A number of different 5-HT₃ receptor antagonists have been disclosed, for example those mentioned in the following patent applications (and hereinafter referred to as the compounds of Group A):

| | | | | |
|---|---|---|---|---|
| AU619731 | AU626614 | AU8658939 | AU8660274 | AU8716591 |
| AU8767121 | EP067770 | EP158265 | EP158532 | EP189002 |
| EP190920 | EP191562 | EP200444 | EP202062 | EP210840 |
| EP212802 | EP214772 | EP219193 | EP220011 | EP221702 |
| EP230718 | EP234872 | EP235878 | EP237281 | EP239321 |
| EP242973 | EP254584 | EP255297 | EP261964 | EP266730 |
| EP266899 | EP276163 | EP287196 | EP289170 | EP291172 |
| EP294292 | EP297651 | EP302699 | EP306148 | EP306323 |
| EP307145 | EP307172 | EP309423 | EP311724 | EP313393 |
| EP315316 | EP315390 | EP317088 | EP322016 | EP323077 |
| EP327307 | EP327335 | EP328200 | EP329932 | EP330788 |
| EP330824 | EP336759 | EP337547 | EP338650 | EP339669 |
| EP339950 | EP339959 | EP344015 | EP345956 | EP350130 |
| EP351385 | EP353983 | EP356098 | EP357417 | EP358903 |
| EP361317 | EP361629 | EP375045 | EP376624 | EP377238 |
| EP378111 | EP381422 | EP385722 | EP387431 | EP392663 |
| EP393766 | EP403261 | EP403882 | EP405784 | EP407137 |
| EP410509 | EP419397 | EP420086 | EP429984 | EP430190 |
| EP436245 | EP454121 | EP456519 | EP457243 | EP458636 |
| EP469449 | EP482939 | EP483836 | EP485962 | EP490263 |
| EP491664 | EP498466 | EP554794 | GB2125398 | GB2145416 |
| GB2152049 | GB2153821 | GB2169292 | GB2192885 | GB2208385 |
| GB2208862 | GB2213816 | GB2216516 | GB2225574 | GB2229182 |
| GB2236528 | GB2236751 | GB2247886 | US4290227 | US4486441 |
| US4657911 | US4789673 | US4816453 | US4822881 | US4826838 |
| US4857517 | US4859683 | US4914207 | US4920219 | US4921982 |
| US4924010 | US4935511 | US4963689 | US4992461 | US4997956 |
| US5030646 | US5063230 | US5116984 | US5137893 | WO8403281 |
| WO8801866 | WO8909217 | WO9006309 | WO9101316 | WO9104738 |
| WO9107402 | WO9112254 | WO9116888 | WO9117161 | WO9205174 |
| WO9206689 | WO9209284 | WO9212149; | | |

The above applications also describe, in relation to the 5-HT₃ receptor antagonists they disclose, both suitable methods for their preparation and doses for their administration.

In UK Patent No. 2153821B there is disclosed, *inter alia,* 1, 2, 3, 9-tetrahydro-9-methyl-3-[(2-methyl-1H-imidazol-1-yl)methyl]-4H-carbazol-4-one, now known as ondansetron, which may be represented by the formula (I) 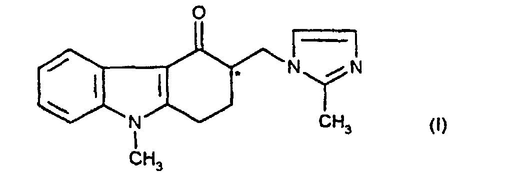 and pharmaceutically acceptable salts, solvates and pharmaceutically acceptable equivalents thereof.

Particular examples of compounds from within Group A include the compounds of Group B, namely:
endo-N-(8-methyl-8-azabicyclo[3.2.1]oct-3-yl)2,3-dihydro-2-oxo-1H-benzimidazole-1-carboxamide (itasetron);
endo-N-(8-methyl-8-azabicyclo[3.2.1]oct-3-yl)2,3-dihydro-3-ethyl-2-oxo-1H-benzimidazole-1-carboxamide (BIMU 1);
endo-8-methyl-8-azabicyclo[3.2.1]oct-3-yl indole-3-carboxylate (tropisetron);
endo-N-(9-methyl-9-azabicyclo[3.3.1]non-3-yl)-1-methylimidazole-3-carboxamide (granisetron);
trans-hexahydro-8-(3-indolylcarbonyloxy)-2,6-methano-2H-quinolizin-3(4H)one (dolasetron), preferably in the form of its mesilate;
endo-5-chloro-2,3-dihydro-2,2-dimethyl-N-(8-methyl-8-azabicyclo[3.2.1]oct-3-yl)-7-benzofuran carboxamide (zatosetron);
4-amino-N-[1-azabicyclo(2.2.2)oct-3-yl]-5-chloro-2-methoxy benzamide (zacopride), more preferably (R) zacopride;
4-[N-(1-azabicyclo[2.2.2]octan-3-(5)-yl)]2-chloro-cis 5a-(s)-9a-(s)-5a,6,7,8,9,9a-hexahydrobenzofurancarboxamide (RG-12915);
4-amino-5-chloro-N-[2-pyrrolidylamethyl)-2,3-dihydrobenzo[b]furan-7-carboxamide (ADR-851);
4-amino-N-[1-azabicyclo[2.2.1]oct-3-yl]-5-chloro-2,3-dihydrobenzo[b]furan-7-carboxamide (ADR-882);
(R)-5-[(1-methyl-3-indolyl)carbonyl]-4,5,6,7-tetrahydro-1H-benzimidazole (YM060);
(±)-N-(1-azabicyclo[2.2.2]oct-3-yl)-6-chloro-4-methyl-3-oxo-3,4-dihydro-2H-1,4-benzoxazine-8-carboxamide (azasetron);
endo-N-(8-methyl-8-azabicyclo[3.2.1]oct-3-yl)-2,3-dihydro-3,3-dimethyl indole-1-carboxamide (BRL 46470); and
2,3,4,5-tetrahydro-5-methyl-2-[(5-methyl-1H-imidazol-4-yl)methyl]-1H-pyrido[4,3-b]indol-1-one (alosetron), preferably as its hydrochloride;
6-fluoro-2,3,4,5-tetrahydro-5-methyl-2-[(5-methyt-1H-imidazol-4-yl)methyl]-1H-pyrido[4,3-b]indol-1-one (lurosetron), in particular in the form of its mesilate dihydrate;
4H-pyrido(3,2,1-jk)carbazol-11(8H)-one, 5,6,9,10-tetrahydro-10-((2-methyl-1H-imidazol-1-yl)methyl)-, (R)- (cilansetron);
benzamide, 4-amino-5-chloro-N-(8-methyl-8-azabicyclo(3.2.1) oct-3-yl)-2-((1-methyl-2-butynyl)oxy)-, monohydrochloride, (3(S)-endo) (E-3620);
pyrido(1,2-a)indol-6(7H)-one, 8,9-dihydro-10-methyl-7-((5- methyl-1H-imidazol-4-yl)methyl)-, monohydrochloride, (+)-(FK-1052);
benzamide, 4-amino-N-1-azabicyclo(2.2.2)oct-3-yl-5-chloro-2-(cyclopropylmethoxy)-, (+/-)- (pancopride); and
7-methoxy-1H-indole-3-carboxylic acid, 8-methyl-8-azabicyclo(3.2.1)oct-3-yl ester.

It will be appreciated by the skilled person that, as a consequence of the use of different chemical naming conventions (e.g. IUPAC, CA), a compound in Group B may be referred to by a different chemical name within the corresponding patent application in Group A.

Other examples of 5-HT₃ receptor antagonists include the compounds of Group C, namely:
1H-Indole, 2,3-dihydro-1-((4,5,6,7-tetrahydro-1H-benzimidazol-5-yl)carbonyl)-, monohydrochloride, (R)-(YM-114);
1H-Benzimidazole, 1-(phenylmethyl)-2-(1-piperazinyl)-(lerisetron);
endo-3,9-dimethyl-3,9-diazabicyclo(3.3.1)non-7-yl-1H-indazole-3-carboxamide dihydrochloride (N-3389);
RS-25259;
endo-8-methyl-8-azabicyclo(3.2.1)oct-3-yl 1-isobutyl-2-oxo-1,2-dihydro-4-quinuclidinecarboxylate (KF-18259);
2-methoxy-4-amino-5-chloro-N-(hexahydro-1H,2,5beta-methano- 3alpha,6alpha-cyclopenta(c)-pyrrol-4alpha-4-(R)-yl) benzamide hydrochloride (SC-52491);
(3aS)-(2-((S)-1-azabicydo(2.2.2)oct-3-yl)-2,3,3a,4,5,6- hexahydro-1H-benz(de)isoquinolin-1-one hydrochloride;
endo-1-Cyclohexyl-N-(8-methyl-8-azabicyclo(3.2.1)octan-3-yl)-4-oxo-1,4-dihydrquinoline-3-carboxamide (mirisetron);
6-amino-5-chloro-1-isopropyl-2-(4-methyl-1-piperazinyl) benzimidazole dimaleate (KB-6933);
(+)-endo-4-(propionyloxy)-6-(4-fluorophenyl)-1-azabicyclo(3.3.1)non-6-ene hydrochloride (GYKI-46903); and
3-(2-(4'-piperonylpiperazinyl)indol)carboxyaldehyde (VA-21 B7).

5-HT₃ receptor antagonists are known to be useful in the treatment of a variety of conditions involving 5-HT₃ receptor-mediated mechanisms, including in particular emesis.

Abnormal involuntary movements or dyskinesias include, *inter alia,* tremor, chorea, myoclonus and tics. In addition to these categories of dyskinesia, there is a particular set of involuntary movements known as tardive dyskinesia or drug-induced tardive dyskinesia.

Tremor is an abnormal involuntary trembling or quivering. It may be characterised as a rhythmic sinusoidal movement of a body part caused by regular rhythmic muscle contractions.

Chorea consists of a continuous flow of irregular, jerky, and explosive movements, that flit from one portion of the body to another in random sequence. Each muscle contraction is brief, often appearing as a fragment of what might have been a normal movement, and quite unpredictable in timing or site.

Myoclonus consists of rapid shock-like muscle jerks, often repetitive and sometimes rhythmic.

Tics are similar to myoclonic jerks in appearance, but are repetitive, stereotyped movements that can be mimicked voluntarily and can be held in check by an effort of will at the expense of mounting inner tension.

Tardive dyskinesia is typically marked by involuntary repetitive movements of the facial, buccal, oral and cervical musculature,

Current medical therapies for dyskinesia, in particular where dyskinesia is the solitary feature of the illness (e.g. benign essential (familial) tremor and benign essential (myoclonus)), include alcohol; benzodiazepines, such as diazepam, lorazepam and clonazepam; and β-adrenergic receptor antagonists, such as propranolol. Other dyskinesias are often a facet of another neurological disease and treatment of the dyskinesia follows that appropriate for the relevant neurological disease. Thus, for example, the treatment of tremor caused by Parkinson's disease may be by dopaminergic agents such as levodapa and antimuscarinic agents such as benzhexol.

Nevertheless, many patients gain slight or even no relief from such agents. Furthermore, the side effect profiles of such drugs severely limit their clinical use. Thus, there is a real need to develop new medicines for the treatment of dyskinesia.

In a preferred aspect the invention therefore provides ondansetron or a pharmaceutically acceptable derivative thereof for use in the treatment of tremor.

Suitable pharmaceutically acceptable salts of ondansetron include acid addition salts formed with inorganic or organic acids (for example hydrochlorides, hydrobromides, sulphates, phosphates, benzoates, naphthoates, hydroxynaphthoates, p-toluenesulphonates, methanesulphonates, sulphamates, ascorbates, tartrates, salicylates, succinates, lactates, glutarates, glutaconates, acetates, tricarballylates, citrates, fumarates and maleates), and solvates (for example hydrates) thereof.

In a preferred embodiment of the present invention ondansetron is employed in the form of its hydrochloride, more preferably its hydrochloride dihydrate.

It will be appreciated by those skilled in the art that ondansetron contains one chiral centre (shown by * in the formula (I)) and that ondansetron therefore exists in the form of optical isomers (i.e. enantiomers). The invention includes all isomers of ondansetron and its pharmaceutically acceptable derivatives, including all tautomeric and optical forms, and mixtures thereof, including racemic mixtures.

Tremor includes rest tremor, postural tremor and intention tremor. Rest tremor may be caused by, for example, Parkinson's disease, post-encephalitic Parkinsonism, drug-induced Parkinsonism and other extrapyramidal diseases. Postural tremor may be caused by, for example, physiological tremor; exaggerated physiological tremor attributable to, for example, thyrotoxicosis, anxiety states, alcohol, drugs (such as, sympathomimetics, anti-depressants and lithium) and heavy metal poisoning (such as mercury); hyperthyroidism, structural brain disease attributable to, for example, severe cerebellar lesions, Wilson's disease and neurosyphilis; and benign essential (familial) tremor. Intention tremor may be caused by, for example, brain stem or cerebellar disease attributable to, for example, multiple sclerosis (MS), spinocerebellar degenerations, vascular disease and tumour.

In a preferred aspect the invention provides ondansetron or a pharmaceutically acceptable derivative thereof for use in the treatment of tremor.

In another preferred aspect the invention provides ondansetron or a pharmaceutically acceptable derivative thereof for use in the treatment of benign essential (familial) tremor.

Benign essential (familial) tremor may present at any age and is often inherited as an autosomal dominant trait. It is characterised by postural tremor of the arms and head, although the jaw, tongue, legs and trunk may also be affected.

In a further preferred aspect the invention provides ondansetron or a pharmaceutically acceptable derivative thereof for use in the treatment of intention tremor.

Within the above aspects and preferred aspects of the invention, the use ondansetron, is especially preferred.

It is to be understood that reference to treatment includes both treatment of established symptoms and prophylactic treatment, unless explicitly stated otherwise.

Conveniently, ondansetron or a pharmaceutically acceptable derivative thereof may be formulated in conventional manner using one or more pharmaceutically acceptable carriers or excipients. Thus ondansetron or a pharmaceutically acceptable derivative thereof may, for example, be formulated for oral, sub-lingual, buccal, parenteral, rectal or intranasal administration, or in a form suitable for administration by inhalation or insufflation (either through the mouth or nose), or in a form suitable for topical administration.

For oral administration the pharmaceutical compositions may take the form of, for example, tablets or capsules prepared by conventional means with pharmaceutically acceptable excipients such as binding agents (e.g. pregelatinised maize starch, polyvinylpyrrolidone or hydroxypropyl methylcellulose); fillers (e.g. lactose, microcrystalline cellulose or calcium phosphate); lubricants (e.g. magnesium stearate, talc or silica); disintegrates (e.g. potato starch or sodium starch glycollate); or wetting agents (e.g. sodium lauryl sulphate). The tablets may be coated by methods well known in the art. Liquid preparations for oral administration may take the form of, for example, solutions, syrups or suspensions, or they may be presented as a dry product for constitution with water or other suitable vehicle before use. Such liquid preparations may be prepared by conventional means with pharmaceutically acceptable additives such as suspending agents (e.g. sorbitol syrup, methyl cellulose or hydrogenated edible fats); emulsifying agents (e.g. lecithin or acacia); non-aqueous vehicles (e.g. almond oil, oily esters or ethyl alcohol); and preservatives (e.g. methyl or propyl-p-hydroxybenzoates or sorbic acid).

For buccal administration the compositions may take the form of tablets or lozenges formulated in conventional manner.

Ondansetron or a pharmaceutically acceptable derivative thereof may be formulated for parenteral administration by injection, conveniently intravenous, intramuscular or subcutaneous injection, for example by bolus injection or continuous intravenous infusion. Formulations for injection may be presented in unit dosage form e.g. in ampoules or in multi-dose containers, optionally with an added preservative.

The compositions for parenteral administration may take such forms as suspensions, solutions or emulsions in oily or aqueous vehicles, and may contain formulatory agents such as suspending, stabilising and/or dispersing agents. Alternatively, the compositions may be in dry form such as a powder, crystalline or freeze-dried solid for constitution with a suitable vehicle, e.g. sterile pyrogen-free water or isotonic saline before use. They may be presented, for example, in sterile ampoules or vials.

Ondansetron or a pharmaceutically acceptable derivative thereof may also be formulated in rectal compositions such as suppositories or retention enemas.

Tablets for sub-lingual administration may be formulated in a conventional manner.

For intranasal administration, or administration by inhalation or insufflation, ondansetron or a pharmaceutically acceptable derivative thereof may be formulated in a conventional manner.

For topical administration the pharmaceutical compositions may be liquids, for example solutions, suspensions or emulsions presented in the form of creams or gels.

In addition to the formulations described previously, ondansetron or a pharmaceutically acceptable derivative thereof may also be formulated as a depot preparation. Such long acting formulations may be administered by implantation (for example subcutaneously, transcutaneously or intramuscularly) or by intramuscular injection. Thus, for example, the compositions may be formulated with suitable polymeric or hydrophobic materials (for example as an emulsion in an acceptable oil) or ion exchange resins, or as sparingly soluble derivatives, for example, as a sparingly soluble salt.

Pharmaceutical formulations of ondansetron or a pharmaceutically acceptable derivative thereof may be prepared, for example, according to UK Patent No. GB 2153821B, European Patent Application Publication No. 276559 or International Patent Application Publication Nos. WO94/27599, WO96/15785 or WO96/15786.

It will be appreciated that the precise therapeutic dose of ondansetron, expressed in the form of its free base, will depend on the age and condition of the patient and the nature of the dyskinesia to be treated, and will be at the ultimate discretion of the attendant physician.

However, in general, effective doses for the treatment of tremor will lie in the range of 0.001 to 500mg, such as 0.01 to 100mg, preferably 0.05 to 50mg, for example 0.5 to 25mg per unit dose, which could be administered in single or divided doses, for example, 1 to 4 times per day.

In a preferred embodiment, effective doses of ondansetron for the treatment of tremor will lie in the range of 0.05 to 100mg, such as 0.1 to 50mg, preferably 0.5 to 25mg, for example 2, 4, 8 or 16mg of ondansetron per unit dose, which could be administered in single or divided doses, for example, 1 to 4 times per day.

The use of ondansetron in the treatment of tremor is supported by the following clinical data.

A placebo-controlled, double blind, crossover study of intravenous ondansetron in twenty patients with cerebellar tremor caused by MS, cerebellar degeneration or drug toxicity was conducted.

Most had tremor based upon cerebellar involvement from MS; three had familial degenerative disorders involving the cerebellum and one had acquired lithium toxicity. The clinical information and prior treatment of the patients is outlined below in Table 1.

Patients were randomly assigned to receive intravenous ondansetron (8mg or a placebo, consisting of normal saline). The drug was given by intravenous bolus injection. Patients were given the alternate agent at a later interval.

The principal outcome measure was the change in manual co-ordination tasks as assessed by spiral copying, word copying or line drawing and the time to complete a nine-hole peg test. Only 12 patients had sufficient co-ordination to complete the nine-hole peg test.

The clinical outcome was measured at baseline and one hour following the infusion.

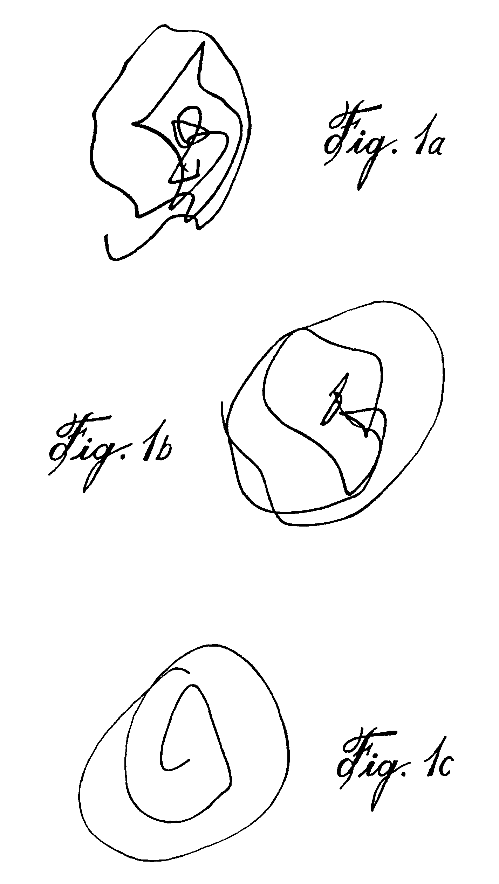

Figures 1 (a), (b) and (c) represent an attempt to copy a spiral by the first mentioned patient in Table 1. Figure 1(a) represents an attempt prior to treatment with ondansetron; figure 1(b) an attempt after receiving placebo; and figure 1 (c) an attempt after receiving ondansetron.

### Results

The patient profile and clinical outcomes are shown in Table 1.

**Table 1**

| Disease | Tremor Type & Clinical Findings | Prior Treatment | Placebo response | Subjective drug response | Spiral Writing placebo | Spiral Writing drug | 9 hole peg-placebo (secs) | 9 hole peg-drug (secs) |
|---|---|---|---|---|---|---|---|---|
| Cd¹ | Intention, postural | Clonazepam | None | ++ | 0 | +++ | 101 | 85 |
| Cd | Intention | | None | +++ | 0 | ++ | 62 | 45 |
| Cd² | Intention | Clonazepam | NT | + (dysarthria) | NT | NT | NT | NT |
| Lithium | Intention/ dysarthria | Clonazepam Valproste | None | 0 | 0 | 0 | 47 | 55 |
| MS | Intention | Clonazepam | None | +++ | 0 | +++ | 84 | 70 |
| MS | Intention | Clonazepam | None | +++ | 0 | +++ | NT | NT |
| MS | Intention | None | None | ++ | 0 | + | 37 | 29 |
| MS | Intention | None | None | + | 0 | + | NT | NT |
| MS | Intention | None | None | ++ | 0 | ++ | NT | NT |
| MS | Intention | None | None | ++ | 0 | + | 140 | 110 |
| MS | Intention | None | None | ++ | 0 | +++ | NT | NT |
| MS | Intention | None | None | 0 | + | + | 78 | 63 |
| MS | Intention | None | None | + | 0 | ++ | NT | NT |
| MS | Intention | None | None | 0 | 0 | 0 | 157 | 155 |
| MS | Intention | None | None | 0 | 0 | + | 65 | 55 |
| MS | Intention | None | None | 0 | 0 | 0 | 65 | 48 |
| MS | Intention | None | None | 0 | 0 | 0 | NT | NT |
| MS | Intention/ dysarthria | None | None | 0 | 0 | + | 160 | 180 |
| MS | Intention/ dysarthria | None | None | + | 0 | ++ | NT | NT |
| MS | Intention | None | NT | 0 | 0 | 0 | 54 | 57 |
| Ratio | | | 0/18 | 12/20 | 1/19 | 13/19 | | |
| Mean | | | | | | | 87±12 | 79±13 |

| | | | | | | | | |
|---|---|---|---|---|---|---|---|---|
| Key Improvement was measured on a singe ordinal scale: 0 = none, + = a little, ++ = moderate, +++ = a lot of improvement, ^{1.} The spiral writing of this patient is shown in Figure 1(a), 1(b) and 1(c); | | | | | | | | |
| ^{2.} The development of dystonia in this patient dissipated enthusiasm to continue in the study; Cd = Cerebellar degeneration. | | | | | | | | |

A favourable clinical response was identified by a panel of three blind observers in 13 of the 20 patients; the same observers identified a response in one placebo-treated patient. The difference was significant in the entire study group (p=0.009; Fisher exact ratio), and the MS subgroup of 16 patients, 13 of whom has a response (p=0.001).

The completion of a timed task, the nine hole peg test, was improved in patients who received ondansetron. The mean time for the completion of this task was 79 ± 13 (standard error) seconds in patients treated with ondansetron and 87 ± 12 seconds in placebo-treated patients, (p=0.04; Mann Whitney; one tailed test for paired data). One further patient with cerebellar degeneration too disabled to complete these tasks (Cd², Table 1), was noticed to have improved speech after treatment with ondansetron.

Patient identification of treatment assignment was correct in 12/20 instances and this was based upon efficacy. None believed placebo to be better (p=0.014; Fisher exact ratio).

The recruitment of patients was biased to select the most severely challenged patients. Most patients required walking assistance. After the study was completed, it was apparent that the best responses were seen in the less disabled patients. Among the patients with MS, a better response was seen in patients with more recent onset ataxia.

Ondansetron was well tolerated and the effect could be sustained in the long term. Withdrawal of the medication resulted in the tremor returning to pre-study levels. Nothing worked well for the tremor in these patients prior to the use of ondansetron.

The effect of intravenous ondansetron could be reproduced with oral ondansetron (8-16mg).

## Claims

1. Use of ondansetron or a pharmaceutically acceptable derivative thereof in the manufacture of a medicament for the treatment of tremor.

2. Use according to claim 1 wherein the tremor is benign essential (familial) tremor.

3. Use according to claim 1 wherein the tremor is intention tremor.

4. Use according to any preceding claim wherein ondansetron is in the form of its hydrochloride or hydrochloride dihydrate.

## Patentansprüche

1. Verwendung von Qndansetron oder eines pharmazeutisch verträglichen Derivats davon zur Herstellung eines Medikaments zur Behandlung von Tremor.

2. Verwendung gemäß Anspruch 1, wobei der Tremor ein hereditärer/essentieller Tremor ist.

3. Verwendung gemäß Anspruch 1, wobei der Tremor ein Intentionstremor ist.

4. Verwendung gemäß einem der vorstehenden Ansprüche, wobei das Ondansetron in der Form seines Hydrochlorids oder Hydrochlorid-Dihydrats vorliegt.

## Revendications

1. Utilisation d'ondansétron ou d'un de ses dérivés pharmaceutiquement acceptables dans la production d'un médicament destiné au traitement des tremblements.

2. Utilisation suivant la revendication 1, dans laquelle les tremblements consistent en tremblements essentiels bénins (familiaux).

3. Utilisation suivant la revendication 1, dans laquelle les tremblements consistent en tremblements intentionnels.

4. Utilisation suivant l'une quelconque des revendications précédentes, dans laquelle l'ondansétron est sous forme de son chlorhydrate ou dihydrate de chlorhydrate.
